Europäisches Patentamt

European Patent Office

Office européen des brevets

(11) Publication number: **0 121 243**
**B1**

# EUROPEAN PATENT SPECIFICATION

(45) Date of publication of patent specification: **23.12.87**

(21) Application number: **84103488.7**

(22) Date of filing: **29.03.84**

(51) Int. Cl.⁴: **G 01 N 33/546,**
**G 01 N 33/569**

(54) **Latex reagent.**

(30) Priority: **01.04.83 JP 57060/83**

(43) Date of publication of application:
**10.10.84 Bulletin 84/41**

(45) Publication of the grant of the patent:
**23.12.87 Bulletin 87/52**

(84) Designated Contracting States:
**BE DE FR GB IT NL**

(56) References cited:
**EP-A-0 054 249**
**EP-A-0 064 275**
**EP-A-0 087 728**
**EP-A-0 153 519**
**US-A-4 533 630**

**PATENTS ABSTRACTS OF JAPAN, vol. 8, no.
68 (P-264) 1505r, 30th March 1984; JP - A - 58
215 558**

(73) Proprietor: **MITSUBISHI CHEMICAL
INDUSTRIES LIMITED
5-2, Marunouchi 2-chome Chiyoda-ku
Tokyo 100 (JP)**
(73) Proprietor: **Ueno, Kazue
Hidase 453
Seki Gifu-ken (JP)**

(72) Inventor: **Ueno, Kazue
Hidase 453
Seki, Gifu-ken (JP)**
Inventor: **Kohno, Hideki
25-13 Tsuchihashi 1-chome Miyamae-ku
Kawasaki Kanagawa-ken (JP)**
Inventor: **Tsutsui, Satoshi
13-13 Tsukimino 7-chome
Yamato Kanagawa-ken (JP)**

(74) Representative: **Patentanwälte TER MEER -
MÜLLER - STEINMEISTER
Mauerkircherstrasse 45
D-8000 München 80 (DE)**

Courier Press, Leamington Spa, England.

(58) References cited:

**CHEMICAL ABSTRACTS, vol. 100, no. 25, 18th June 1984, page 156, no. 204591m, Columbus, Ohio, US; T. YOKOTA et al.: "A new immunoassay of cholera toxin with stable polystyrene latex particles"**

**INFECTION AND IMMUNITY, vol. 20, no. 2, May 1978, pages 526-529, American Society of Microbiology, Washington, DC, US; T.W. CHANG et al.: "Clindamycin-induced enterocolitis in hamsters as a model of pseudomembranous colitis in patients"**

## Description

Technical field of the invention

This invention relates to a toxin-detecting latex reagent.

Background of the invention

As a result of increased clinical use of varieties of antibiotic chemotherapeutic agents in recent years, symptoms primarily comprising diarrhoea are today being reported in large numbers.

Such diarrhoeal symptoms are typically caused by pseudomembranous colitis. Virus theories and allergy theories, have been propounded as the mechanism of occurrence of grave colitis of this type. However, as a result of the study on enterotoxin, an exotoxin produced by enterobacteria, it has recently come to be thought that the principal cause of such colitis is the proliferation of a certain toxogenic bacteria induced by the change in the enteric bacterioflora caused by administration of chemotherapeutic agents.

For instance, a study by Ueno et al on D-1 toxin and D-2 toxin, both of which are produced by Clostridium difficile, a common anaerobic enterobacterium, was published in Biochemistry International, Vol. 2, No. 6, pp. 629—635, 1981. According to this report, D-1 and D-2 impair permeability of cells, injure HeLa cells and are a direct cause of pseudomembranous colitis.

Clinically, pseudomembranous colitis causes prolonged symptom of watery diarrhoea containing mucus, leading to general hyposthenia and sometimes resulting in death.

In the clinical diagnosis of this disease, an S-shape colonoscope is used, biopsy of the rectum is carried out, and detection of pathogenic bacteria from faeces is required. These diagnostic tests require sophisticated techniques and consume much time.

We pursued an extensive study to find ways for diagnosing this disease more simply and quickly with higher accuracy. We first obtained an immunoglobulin fraction containing IgG, and provided a toxin-detecting latex reagent by sensitizing latex particles with said immunoglobulin fraction. We further proceeded with the study in order to obtain a further improved latex reagent furnished with improved storage stability. As a result of this study, we reached this invention.

Disclosure of the invention

The gist of this invention resides in a toxin-detecting latex reagent obtained by isolating an immunoglobulin fraction containing IgG from antisera against a toxin of an enterobacterium, sensitizing latex particles with said fraction, and treating said sensitized latex particles with a protein solution.

The toxins to be detected by the reagent of this invention include toxins produced by *Clostridium difficile*, *Clostridium perfrigens*, *Vibrio cholerae*, *Shigella dysenterieae*, *Escherichia coli* and *enteritis Vibrio*.

The method for obtaining the antisera against these enterobacterium toxins is explained with respect to Clostridium difficile as an example in the following.

D-1 and D-2 toxins are obtained by the method of Ueno et al as described in the above-cited Biochemistry International.

Strains of Clostridium difficile, an anaerobic bacterium, are collected from faeces of a pseudomembranous colitis patient and are anaerobically cultured in a culture medium containing 5% brain-heart infusion and 1% protease-pepton so as to produce D-1 and D-2 toxins. The medium is incubated at 37°C for 48 hours and thereafter, centrifuged at 8,000×G for 30 minutes. The supernatant is filtered with a membrane filter with 0.45 μm pores.

To the filtrate, mercaptoethanol is added so that the concentration thereof is 5 mM and the toxins are concentrated by salting out with ammonium sulfate, which is added so that the concentration thereof is 70% of saturation. The concentrated toxins are purified by chromatography with a column of AcA (an adsorbent, product of LKB, Inc.), and pure D-1 (estimated molecular weight 600,000) and D-2 (estimated molecular weight 48,000) are obtained.

Of course, only one of D-1 and D-2 can be separated and used as the starting material for preparation of the antiserum, which is explained below.

Laboratory animals (rabbit, guinea-pig or goat) are immunized with the toxins per se, or the toxins detoxicated with formaldehyde or glutalaldehyde, for instance, and an antiserum which has high specificity against D-1 and D-2 toxins is obtained. The antibody values of this antiserum can be confirmed by the Ouchterlony test (diffusion test in two dimensions) and electrophoresis.

An immunoglobulin fraction containing IgG (referred to as "IgG fraction" hereinafter) is collected from the antiserum. The method for obtaining this fraction from the obtained antiserum includes salting-out from an ammonium sulfate solution (when collection of a fraction containing IgG is intended), or fractionation of IgG with a weakly acidic ion exchange resin (when collection of a fraction containing IgG only is intended).

The thus obtained IgG fraction is dissolved in a buffer solution and vigorously agitated together with latex particles to sensitize the latex particles with IgG. The usable latexes include those of organic macromolecular substances such as polystyrene, styrene-butadiene copolymer, poly(acrylic ester) and poly(methacrylic ester). These latexes are well known and commercially available.

Further, the desired sensitized latexes can be obtained by chemically combining carboxylic acid-, amine- and amide-type latexes containing functional substituents with the IgG.

3

The thus obtained sensitized latex reagent (a suspension) of this invention is treated with a protein solution in order to improve the storage stability and accuracy in detection of toxins. Proteins soluble in water and a buffer solution can be used for this purpose without restriction, although proteins such as albumin, globulin and fibrinogen are usually used for this purpose, and synthesized polypeptides can also be used. The concentration of the protein solution used is usually 0.01%—2%, preferably 0.1—0.5%, the pH of the solution is 5—10, preferably around 8. The buffer solution usually used is phosphoric acid type buffer or boric acid type buffer. The treatment is usually carried out at room temperature.

The treatment with a protein solution is, for instance, conducted as follows. The sensitized latex solution as described above is centrifuged and the supernatant is removed. The obtained precipitate is agitated with the above-mentioned protein solution for several minutes to several hours. The suspension is further centrifuged and the supernatant is removed. The collected precipitate is suspended in water or a buffer solution and thus the intended latex reagent is obtained.

The latex reagent may contain additives such as stabilizing agents, antiseptics, chelating agents and surfactants. Usable buffering agents include glycine buffer, phosphoric acid buffer, citric acid buffer, barbital buffer, boric acid buffer, tris-[tris(hydroxymethyl)aminomethane]-HCl buffer, tris-malate buffer and ammonia buffer.

The method for detecting toxins produced by Clostridium difficile in faeces using the thus obtained latex reagent is as follows.

Watery or hard faces collected from a pseudomembranous colitis patient is extracted with a 0.9% NaCl solution or a phosphoric acid buffer solution containing 0.9% NaCl, and the extract is subjected to a clinical test.

When the above described latex reagent is added to the extract, coagulation of the latex occurs by the polygenic antigen-antibody reaction of the IgG on the surface of the latex particles and D-1 and D-2 toxins in the extract. The degree of coagulation of the latex is observed macroscopically on a slide glass and detection of toxins of colitis patients can be semi-quantitatively conducted. Diagnosis of pseudomembranous colitis from Clostridium difficile can be carried out far more rapidly and with higher accuracy than the conventional methods.

If the above described latex reagent is used with an automated immunity diagnosis apparatus, a further rapid quantitative determination is possible.

Description of the preferred embodiments
The invention is now explained by way of working examples.

Example 1
D-1 and D-2 toxins which had been collected and purified by the method of Ueno et al (Biochemistry International, Vol. 2, No. 6, pp. 629—635, (1981)) were treated with a 0.4% formaldehyde solution at 37°C for 72 hours for detoxication. The detoxicated toxins were prepared into about 500 µg/ml of a solution in the physiological salt solution. The solution was well admixed with Freund's complete adjuvant (product of Difico) until the mixture became viscous. This toxin preparation was administered to a rabbit. After the first administration, the rabbit was further immunized every other week four times in all. Two weeks after the last immunization, blood was collected from the rabbit and an antiserum was obtained. The antibody value was determined by the Ouchterlony test and immunity electrophoresis.

Preparation of latex reagent
The obtained antiserum was added to a 38% ammonium sulfate solution, and the formed precipitate (a fraction containing IgG, i.e., γ-globulin) was collected. This fraction was partially purified by dialysis and used for preparation of the latex reagent. That is, the IgG fraction, which was deposited from the antiserum against D-1 and D-2 by salting out with ammonium sulfate (38% of saturation) was dissolved in an 0.2M boric acid solution (pH 8.0) so that the concentration was 800—1500 antibodies par one particle of the latex to be used. A latex-boric acid buffer solution (pH 8) was prepared using a polystyrene latex having particle diameter of 0.48 µm (product of Dow Chemical Corp.) and a boric acid buffer solution. The above-mentioned IgG solution was mixed with an equal amount of said latex-boric acid buffer solution and well agitated so as to cause satisfactory sensitization. The mixture was centrifuged and the supernatant was removed. The thus obtained precipitate was treated with the boric acid buffer solution containing 0.2% bovine serum albumin at room temperature for 15 minutes under agitation. The mixture was further centrifuged and the supernatant was removed and the obtained latex particles were suspended in a boric acid buffer solution for use. This latex reagent was stable for not less than 6 months and retained stable accuracy in clinical tests.

Comparative Example 1
The latex reagent which was obtained in accordance with the procedure of Example 1 but was not treated with the bovine serum albumin developed coagulation of latex particles in 24 hours and it could no longer be used for clinical tests.

Example 2

Faeces of a colitis patient was added to a 10% physiological salt solution and was vigorously agitated. The mixture was centrifuged at 3,000 rpm for 10 minutes and the supernatant was collected and filtered with a 0.45 µm millipore filter. The filtrate was used as a sample.

One hundred (100) µl physiological salt solution containing 0.2% bovine serum albumin was dropped on a slide glass, and 50 µl of the above described sample solution was added thereto. After the above two were well mixed, 30 µl of the latex reagent was added and mixed. After a few minutes, formation of latex clots was macroscopically observed.

When the extract of faeces of a normal person was used as the sample, the latex particles remained in the state of suspension and no coagulation thereof was observed. In contrast, the sample from faeces of a pseudomembranous colitis patient caused distinct coagulation of latex particles and the degree of the coagulation was proportional to the concentration of the toxins.

Example 3

The same clinical sample solution as used in Example 2 was used for the test by means of an optical immunological diagnosis system ("LPIA (Latex photometric Immunoassay)" manufactured by Mitsubishi Chemical Industries Ltd.). Fifty (50) µl of the sample solution was taken into a reaction cuvette and 200 µl of a buffer solution containing 0.1% bovine serum albumin was added thereto to make 250 µl. Fifty (50) µl of the latex reagent (1% latex solution) prepared in Example 1 was automatically fed in the reaction cuvette together with 200 µl of a physiological salt solution containing 0.1% bovine serum albumin. Thus change in turbidity due to the antigen-antibody reaction in the latex reaction mixture (500 µl in total). Reaction was monitored for 60 seconds at 940 nm wavelength. A calibration curve concerning the degree of the change with respect to the standard samples had been prepared. The amount of the toxins of Clostridium difficile in the sample was accurately determined.

Although the invention has been described in detail with respect to a few specific examples pertaining to Clostridium difficile, those skilled in the art who read the description will understand that latex reagents for detecting toxins of enterobacteria other than Clostridium difficile can be obtained in accordance with the knowledge of clinical biochemistry.

**Claims**

1. A toxin-detecting latex reagent comprising latex particles which are sensitized with Immunoglobulin G (IgG) which has been obtained from an antiserum against a toxin of an enterobacterium, and which are treated with a protein solution.

2. The toxin-detecting latex reagent according to Claim 1, wherein the latex particles sensitized with IgG are treated with a solution of a protein selected from albumin, globulin and fibrinogen.

3. The toxin-detecting latex reagent according to Claim 1, wherein the latex is one selected from polystyrene latex, styrene-butadiene copolymer latex, poly(acrylic acid ester) latex, and poly(methacrylic acid ester) latex.

4. A process for preparing a toxin-detecting latex reagent comprising isolating an IgG-containing fraction from an antiserum against a resin of an enterobacterium, sensitizing the latex particles with said IgG, and treating said sensitized latex particles with a protein solution.

5. The process for preparing the toxin-detecting latex reagent according to Claim 4, wherein the latex is one selected from polystyrene latex, styrene-butadiene copolymer latex, poly(acrylic acid ester) latex, and poly(methacrylic acid ester) latex.

6. The process for preparing the toxin-detecting latex reagent according to Claim 4, wherein the latex solution sensitized with IgG are treated with a solution of a protein selected from albumin, globulin and fibrinogen.

**Patentansprüche**

1. Latexreagens zum Nachweis von Toxinen, enthaltend Latexteilchen, die mit Immunoglobulin G (IgG) sensibilisiert sind, das aus einem Antiserum gegen ein Toxin eines Enterobakteriums erhalten wurde, und mit einer Proteinlösung behandelt sind.

2. Latexreagens zum Nachweis von Toxinen nach Anspruch 1, dadurch gekennzeichnet, daß die mit IgG sensibilisierten Latexteilchen mit einer Lösung eines Proteins aus der Albumin, Globulin und Fibrinogen umfassenden Gruppe behandelt worden sind.

3. Latexreagens zum Nachweis von Toxinen nach Anspruch 1, dadurch gekennzeichnet, daß der Latex aus der Polystyrollatex, Styrol-Butadien-Copolymerlatex, Poly(acrylsäureester)latex und Poly(methacrylsäureester)latex umfassenden Gruppe gewählt ist.

4. Verfahren zur Herstellung eines Latexreagens zum Nachweis von Toxinen, dadurch gekennzeichnet, daß man eine IgG-haltige Fraktion aus einem Antiserum gegen ein Gift eines Enterobakteriums isoliert, die Latexteilchen mit dem IgG sensibilisiert und die sensibilisierten Latexteilchen mit einer Proteinlösung behandelt.

5. Verfahren zur Herstellung des Latexreagens zum Nachweis von Toxinen nach Anspruch 4, dadurch gekennzeichnet, daß man als Latex einen Latex aus der Polystyrollatex, Styrol-Butadien-Copolymerlatex, Poly(acryläureester)latex und Poly(methacrylsäureester)latex umfassenden Gruppe einsetzt.

6. Verfahren zur Herstellung des Latexreagens zum Nachweis von Toxinen nach Anspruch 4, dadurch gekennzeichnet, daß man die mit dem IgG sensibilisierte Latexlösung mit einer Lösung eines Proteins aus der Albumin, Globulin und Fibrinogen umfassenden Gruppe behandelt.

**Revendications**

1. Un réactif au latex de détection de toxine comprenant des particules de latex qui sont sensibilisées avec l'immunoglobuline G (IgG) qui a été obtenue à partir d'un antisérum contre une toxine d'une entérobactérie et qui sont traitées avec une solution de protéine.

2. Le réactif au latex de détection de toxine selon la revendication 1, dans lequel les particules de latex sensibilisées avec l'IgG sont traitées avec une solution d'une protéine choisie parmi l'albumine, la globuline et le fibrinogène.

3. Le réactif au latex de détection de toxine selon la revendication 1, dans lequel le latex est choisi parmi un latex de polystyrène, un latex de copolymère de styrène-butadiène, un latex de poly(ester d'acide acrylique) et un latex de poly(ester d'acide méthacrylique).

4. Un procédé pour la préparation d'un réactif au latex de détection de toxine comprenant l'isolement d'une fraction contenant de l'IgG, d'un antisérum contre une toxine d'une entérobactérie, la sensibilisation des particules de latex avec ladite IgG et le traitement desdites particules de latex sensibilisées avec une solution de protéine.

5. Le procédé pour la préparation du réactif au latex de détection de toxine selon la revendication 4, dans lequel le latex est choisi parmi un latex de polystyrène, un latex de copolymère de styrène-butadiène, un latex de poly(ester d'acide arylique) et un latex de poly(ester d'acide méthacrylique).

6. Le procédé pour la préparation du réactif au latex de détection de toxine selon la revendication 4, dans lequel la solution de latex sensibilisée avec l'IgG est traitée avec une solution d'une protéine choisie parmi l'albumine, lu globuline et le fibrinogène.